# EUROPEAN PATENT SPECIFICATION

(11) **EP 3 744 852 B1**
(45) Date of publication and mention of the grant of the patent: **28.06.2023**
(21) Application number: 20175636.8
(22) Date of filing: 20.05.2020
(51) Int. Cl.: C12P 5/02, C12M 1/107, C12M 1/113

(54) **IMPROVED PROCESS AND SYSTEM FOR THE TREATMENT OF ORGANIC MATERIAL**
VERFAHREN UND SYSTEM ZUR BEHANDLUNG VON ORGANISCHEM MATERIAL
PROCÉDÉ ET SYSTÈME POUR LE TRAITEMENT D'UN MATÉRIAU ORGANIQUE

(30) Priority: 24.05.2019 EP 19176475
(43) Date of publication of application: 02.12.2020
(73) Proprietor: B.A.T. Services, 9051 Sint-Denijs-Westrem (BE)
(72) Inventor: De Lathauwer, Bart, 9051 Sint-Denijs-Westrem (BE)
(74) Representative: De Clercq & Partners

(56) References cited:
- EP-A1- 2 135 938
- EP-A1- 3 415 472

## Description

### TECHNICAL FIELD

The present invention concerns an improved process for the anaerobic digestion of organic material, in particular organic material with high dry matter content, and to a system for the actuation of said process.

### BACKGROUND

In recent years, anaerobic digestion has emerged as a valuable technology for the management of organic waste. Anaerobic digestion of organic substrates allows to produce biogas from e.g. agricultural and industrial products and by-products and from household waste. The biogas can be used for the production of electricity, heat or another form of energy. In addition, the initial organic substrate is converted into a partially mineralized product referred to as digestate that contains a higher percentage of nutrients with respect to the initial substrate. The digestate can have an agronomic value comparable to that of chemical fertilizers.

However, the efficiency of current anaerobic digestion processes is often insufficient allowing only the *in situ* use of the produced biogas. Anaerobic digestion plants face the problem of bacterial washout in that part of the bacterial biomass that is responsible for the fermentation is removed from the fermenter with the digestate. The bacterial density largely determines the fermentation speed of the organic substrate and the related biogas production. Many anaerobic digestion plants also largely use "noble" organic substrates like agricultural foodstuff because the digestion of many organic substrates, such as e.g. fibrous-rich substrates, is too low. In general, simple carbohydrates are easiest to digest whereas large molecules are more difficult to digest. Despite its agronomic value, the current management practice of digestate is restricted to its utilization for *in situ* land application either as fertilizer or soil improver due to its high water content which makes it difficult to store, handle and transport, or complex and/or costly processes are required to treat the digestate to be used as fertilizer.

The patent document EP 2 135 938 A1 (TRABATTONI, S. 23.12.2009) discloses:
a process for the treatment of organic material comprising the following steps:
   (a) hydrolyzing the organic material by biological hydrolysis,
   (b) anaerobic digestion of the hydrolyzed organic material in at least two fermenters (acetogenic fermenter and methanogenic fermenter) into biogas and digestate,
   (c) separating the digestate into a liquid fraction and a solid fraction by mechanical means (first flow and second flow),
   (d) recycling the solid fraction of the digestate to the biological hydrolysis (first flow),
   (e) separating the liquid fraction of the digestate into a filtrate and a retentate containing bacterial biomass by filtration means (second flow),
   (g) concentrating the filtrate by dehydration (second flow), and
   (h) recycling of the dehydration water from step (g) to the hydrolysis step (a) for diluting the organic material (second flow),
as well as
a system for the treatment of organic material comprising:
   a) one or more hydrolysis units for hydrolyzing the organic material by biological hydrolysis,
   b) at least two anaerobic fermenters (acetogenic fermenter and methanogenic fermenter) for the production of biogas and digestate by fermentation of the hydrolyzed organic material, said acetogenic fermenter being fed with the hydrolyzed organic material and the methanogenic fermenter comprising at least two output conduits from which the biogas and the digestate are discharged,
   c) a separator connected to the fermenter via the output conduit and receiving the digestate, said separator being capable of separating the digestate into a liquid fraction and a solid fraction,
   d) a conduit allowing said solid fraction of the digestate to flow out of said separator and to be returned to the hydrolysis unit for hydrolyzing the organic material by biological hydrolysis,
   e) a filtration system for separating the liquid fraction of the digestate into a filtrate and a retentate, said filtration system comprising a membrane through which the liquid fraction of the digestate is filtered to obtain a filtrate and a retentate containing bacterial biomass,
   g) a dehydration device for concentrating the filtrate, wherein said dehydration device is connected to the filtration system through a conduit allowing the filtrate to flow out of the filtration system,
   h) a conduit allowing the dehydration water from the dehydration device to recycle to the hydrolysis unit.

The patent document EP 3 415 472 A1 (MURGIA, I., 19.12.2018) discloses:
a process for the treatment of organic material comprising the following steps:
   (a) hydrolyzing the organic material,
   (b) anaerobic digestion of the hydrolyzed organic material in a single fermenter into biogas and digestate,
   (c) separating the digestate into a liquid fraction and a solid fraction by mechanical means,
   (e) separating the liquid fraction of the digestate into a filtrate and a retentate containing bacterial biomass by filtration means,
   (f) recycling the retentate containing bacterial biomass to the fermenter,
as well as
a system for the treatment of organic material comprising:
   a) one or more hydrolysis units for hydrolyzing the organic material,
   b) a single anaerobic fermenter for the production of biogas and digestate by fermentation of the hydrolyzed organic material, said fermenter being fed with the hydrolyzed organic material and comprising at least two output conduits from which the biogas and the digestate are discharged,
   c) a separator connected to the fermenter via the output conduit and receiving the digestate, said separator being capable of separating the digestate into a liquid fraction and a solid fraction,
   e) a filtration system for separating the liquid fraction of the digestate into a filtrate and a retentate, said filtration system comprising a membrane through which the liquid fraction of the digestate is filtered to obtain a filtrate and a retentate containing bacterial biomass,
   f) a conduit allowing the retentate to flow out of said filtration system and to be returned to the anaerobic fermenter.

Facing the problem of proper waste management and the demand for renewable energy, there is a need for more efficient anaerobic digestion processes, in particular anaerobic digestion processes with an improved degradation efficiency and enhanced biogas yield, and which produce digestate of increased quality.

### SUMMARY

The present invention relates to a process and a system for the treatment of organic substrates by anaerobic digestion with improved degradation efficiency, thereby increasing biogas production with the same organic load. The improved degradation efficiency is obtained by pre-treatment of the organic substrate by four hydrolysis steps, which accelerates the rate-limiting hydrolysis step of anaerobic digestion, and by management of the digestate encompassing the separation of the digestate in multiple phases, and recycling of an undigested fraction for pre-treatment by thermal hydrolysis and anaerobic digestion and recycling of the fermenting bacterial biomass to the fermenter. The present invention further allows an efficient recovery of nutrients from the digestate and the production of a dry biofertilizer.

The present invention is in particular captured by the below embodiments:

A process for the treatment of organic material, comprising the following steps:
(a) hydrolyzing the organic material (101) by mechanical hydrolysis, chemical hydrolysis, thermal hydrolysis and biological hydrolysis;
(b) anaerobic digestion of the hydrolyzed organic material (113) in a single fermenter (50) into biogas (117) and digestate (115);
(c) separating the digestate (115) into a liquid fraction (119) and a solid fraction (118) by mechanical means (60);
(d) recycling the solid fraction (118) of the digestate to the thermal hydrolysis;
(e) separating the liquid fraction (119) of the digestate into a filtrate (123) and a retentate (121) containing bacterial biomass by filtration means (70);
(f) recycling the retentate (121) containing bacterial biomass to the fermenter (50);
(g) concentrating the filtrate(123) by dehydration; and
(h) recycling of the dehydration water (127) from step (g) to the hydrolysis step (a) for diluting the organic material (101.

Said process, wherein said process further comprises a step (i) of drying the concentrated filtrate (125).

Said process, wherein the organic material (101) is hydrolyzed by mechanical hydrolysis, chemical hydrolysis, thermal hydrolysis and biological hydrolysis, in that order.

Said process, wherein an alkali reagent, preferably lime (CaO), is used for said chemical hydrolysis.

Said process, wherein said thermal hydrolysis is conducted at a temperature above 70°C for at least 1 hour.

Said process, wherein said biological hydrolysis is conducted by microbial organisms present in the organic material (101).

Said process, wherein said separation step (c) comprises the centrifugation of the digestate or wherein a parabolic screen is used for said separation step (c), preferably a parabolic screen is used.

Said process, wherein the filtrate (123) is concentrated in step (g) by evaporation.

A system (100) for the treatment of organic material (101), comprising:
a) one or more hydrolysis units (10, 20, 30, 40) for hydrolyzing the organic material (101) by mechanical hydrolysis, chemical hydrolysis, thermal hydrolysis and biological hydrolysis;
b) a single anaerobic fermenter (50) for the production of biogas (117) and digestate (115) by fermentation of the hydrolyzed organic material (113), said fermenter being fed with the hydrolyzed organic material (113) and comprising at least two output conduits from which the biogas (117) and the digestate (115) are discharged;
c) a separator (60) connected to the fermenter via the output conduit and receiving the digestate (115), said separator (60) being capable of separating the digestate (115) into a liquid fraction (119) and a solid fraction (118);
d) a conduit allowing said solid fraction of the digestate (118) to flow out of said separator (60) and to be returned to the hydrolysis unit for hydrolyzing the organic material (101) by thermal hydrolysis (30);
e) a filtration system (70) for separating the liquid fraction (119) of the digestate into a filtrate (123) and a retentate (121), said filtration system (70) comprising a membrane through which the liquid fraction of the digestate (119) is filtered to obtain a filtrate (123) and a retentate (121) containing bacterial biomass;
f) a conduit allowing the retentate (121) to flow out of said filtration system (70) and to be returned to the anaerobic fermenter (50);
g) a dehydration device (80) for concentrating the filtrate (123), wherein said dehydration device (80) is connected to the filtration system (70) through a conduit allowing the filtrate (123) to flow out of the filtration system (70);
h) a conduit allowing the dehydration water (127) from the dehydration device (80) to recycle to the hydrolysis unit (20).

Said system further comprising:
i) a dryer (90) for drying the concentrated filtrate (125).

Said system, wherein said system comprises a hydrolysis unit for mechanical hydrolysis (10), a hydrolysis unit for chemical hydrolysis (20), a hydrolysis unit for thermal hydrolysis (30) and a hydrolysis unit for biological hydrolysis (40), wherein said hydrolysis units are placed in series in that order.

Said system, wherein said separator (60) is a centrifuge or a parabolic screen, preferably a parabolic screen.

Said system, wherein said filtration system (70) is an ultrafiltration system.

Said system, wherein said dehydration device (80) is an evaporator.

### DESCRIPTION OF THE FIGURES

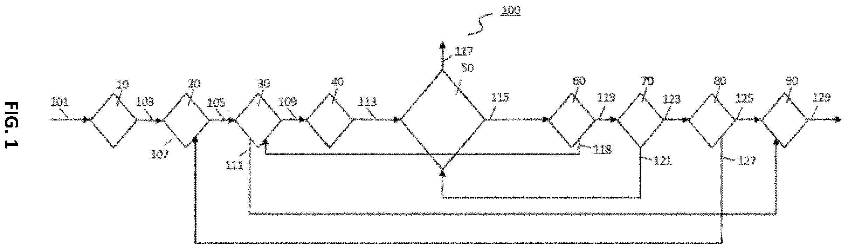

**Fig. 1** shows a schematic of a system (100) and method according to an embodiment of the invention.

### DETAILED DESCRIPTION

The scope of the present invention can only be limited by the relevant claims.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. The preferred methods and materials will now be described.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

As used herein, the singular forms "a," "an" and "the" include both singular and plural referents, unless the context clearly indicates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein. This applies to numerical ranges irrespective of whether they are introduced by the expression "from... to..." or the expression "between... and..." or another expression.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The terms "organic material", "organic substrate" and "organic feed" are used interchangeably herein and refer to products, by-products, and residues (including waste streams) from agriculture, forestry and related industries, as well as industrial and household waste, or a mixture thereof. The term "biomass" as used herein refers to the biodegradable fraction of the organic material. In addition to said biomass, the organic material may further contain e.g. nutrients and water.

Preferably, the organic material (101) described herein has a biodegradable organic matter content or biomass content above 50%, preferably above 60% or 70%, more preferably above 75%, even more preferably above 80%, such as above 85%, above 90% or even above 95% of the dry matter content.

The organic material (101) may be wet (i.e. liquid), dry (i.e. solid) or semi-solid. The processes and systems described herein are particularly useful for the treatment of organic material (101) with a high dry material content, which is the weight of the organic material after the moisture has been extracted. The dry material content or dry matter content can be determined by drying a sample in an oven until a constant weight is obtained. In embodiments, the organic material has a dry matter content above 50% (w/w), preferably above 60 % (w/w) or 70% (w/w), more preferably above 75 % (w/w), even more preferably above 80% (w/w) such as above 85% (w/w), above 90% or even above 95% (w/w).

### Pre-treatment - hydrolysis

Before the organic material (101) is introduced into the fermenter (50) for anaerobic digestion, it may undergo one or more pre-treatments steps. The purpose of such pre-treatment may be to mix different feedstock, to liquefy the organic substrate, to remove undesirable materials such as large items, inert, non-biodegradable materials (e.g. plastic, glass) (separation), or pathogenic micro-organisms (pasteurization, sterilization), to allow a more effective digestion (e.g. hydrolysis) and an increased biogas production and/or a better digestate quality. A great variety of pre-treatment processes are available, the selection of which being dependent amongst others on the feedstock.

In certain embodiments, in particular when using feedstock with a dry matter content above 50%, the organic material or partially hydrolyzed organic material, in particular mechanically hydrolyzed organic material (103), is mixed with water (127), preferably recycled process water. Said mixing preferably takes place during hydrolysis of the organic material. Mixing with water liquefies the organic substrate, which may render it more accessible for certain hydrolytic processes and/or anaerobic digestion. An amount of water may be mixed with the organic matter or partially hydrolyzed organic matter to obtain a diluted feedstock with a preestablished moisture content.

Pre-treatment by hydrolysis enhances the anaerobic digestion. As used herein, the term "hydrolysis" refers to any technique that makes the biomass more accessible for anaerobic digestion. Typically, hydrolysis encompasses the breakdown of the organic material (101) into smaller, fermentable constituents that become available for the bacteria in the fermenter (50).

Hydrolysis of the organic material (101) is obtained by mechanical, thermal, chemical and biological techniques.

In embodiments, the organic material (101) is hydrolyzed by mechanical hydrolysis, chemical hydrolysis, thermal hydrolysis and biological hydrolysis in any order or in that order.

Mechanical hydrolysis involves all types of milling. During mechanical hydrolysis, particle size of the organic substrate is reduced by physical forces (e.g. shearing, compressive forces) resulting in a material that is more susceptible to subsequent hydrolysis steps and/or anaerobic digestion. In embodiments, the organic material is processed by mechanical hydrolysis to a particle size of 8 mm or less, preferably 6 mm or less, more preferably 5 mm or less. Non-limiting examples of suitable means for mechanical hydrolysis include a grinder, a hammer mill, a ball mill, a screw press.

Chemical hydrolysis is used to achieve the (further) destruction of organic material by means of chemicals such strong acids, alkalis or oxidants (e.g. ozonation). A chemical can be selected based on the composition of the feedstock. In embodiments, an alkali reagent is used for chemical hydrolysis (i.e. alkaline hydrolysis). Alkali hydrolysis is particularly useful for combining with subsequent biological hydrolysis to ensure optimal conditions for the micro-organisms. Non-limiting examples of alkali reagents include lime (CaO/Ca(OH)2), NaOH and Na₂CO₃.

Thermal hydrolysis of biomass refers to the treatment of the organic material at a high temperature to solubilize a major part of the particulate matter and/or to convert the organic matter into easily soluble, biodegradable matter. Thermal hydrolysis also effects hygienization (i.e. reducing the content of microorganisms, especially pathogenic microorganisms) of the organic material before it is introduced into the fermenter. Thermal hydrolysis can take place at various temperatures ranging from about 50°C to about 250°C. In embodiments, thermal hydrolysis is conducted at a temperature above 70°C, preferably at a temperature between 70°C and 100°C, for at least 1 hour. The preferred times for this thermal hydrolysis depend inversely on the temperature employed (i.e. a same degree of degradation may be obtained for a thermal treatment at high temperature or longer treatment time at lower temperatures), and further on e.g. the composition of the feedstock. In embodiments, thermal hydrolysis is conducted at a temperature between 75°C and 95°C for between 6 and 24 hours, more preferably at a temperature between 80°C and 90°C for between 9 and 18 hours, such as at about 85°C for about 12 hours. The heat for said thermal hydrolysis may be provided by the system, more particularly residual heat from engines in the system.

Biological hydrolysis encompasses the microbial breakdown of the (partially hydrolyzed) organic material under aerobic or anaerobic conditions to improve the availability of organic constituents for anaerobic digestion. These micro-organisms may be naturally present in the biomass, or they can be added to the (partially hydrolyzed) organic material. In embodiments, the biological hydrolysis of the (partially hydrolyzed) organic material is facilitated by biological processes catalyzed by microbial organisms, including bacteria and fungi, present in the organic material. The retention time of the (partially hydrolyzed) biomass in the hydrolysis unit for biological hydrolysis is typically between 1 and 7 days, preferably between 1 and 6 days, more preferably between 1 and 4 days, even more preferably between 24 hours and 72 hours or between 24 hours and 48 hours. The hydrolysis unit (40) for biological hydrolysis may be cooled.

Hydrolysis is carried out in a hydrolysis unit, which can take various forms such as a conical form. The content of the hydrolysis unit is preferably intermittently or continuously mixed. The hydrolysis unit comprises an inlet for receiving the (partially hydrolyzed) organic material and an outlet for discharging the hydrolyzed organic material. The hydrolysis unit may comprise an additional outlet for discharging material that has deposited.

The system described herein may comprise different hydrolysis units that are sequentially connected to each other, e.g. a first hydrolysis unit (10) for mechanical hydrolysis of the organic material (101), a second hydrolysis unit (20) for chemical hydrolysis of the mechanically hydrolyzed organic material (103), a third hydrolysis unit (30) for the thermal hydrolysis of the mechanically and chemically hydrolyzed organic material (105) and a fourth hydrolysis unit (40) for the biological hydrolysis of the mechanically, chemically and thermal hydrolyzed organic material (109).

### Fermentation or anaerobic digestion

The terms "fermentation", "fermenting" and "anaerobic digestion" are used herein as synonyms and refer to a microbial process that takes place in the absence of oxygen (i.e. anaerobically) in which biomass is converted into simpler products such as methane (CH₄), carbon dioxide (CO₂), H₂ and H₂O.

The microorganisms used in a fermentation process may be a combination of various kinds of microorganisms such as thermophilic or mesophilic bacteria. In embodiments, mesophilic bacteria are used.

Fermentation typically occurs at temperatures of between 15 °C and 65 °C, depending on the microorganisms involved. Temperature ranges that provide suitable digestion conditions for mesophilic bacteria may range between 20°C and 45°C, preferably between 37°C and 41°C such as at about 40°C. Temperature ranges that provide suitable digestion conditions for thermophilic bacteria may range between 50°C and 70°C, preferably between 50°C and 52°C. The residence time of the organic material in the fermenter varies amongst others with the composition of the organic material and the process temperature and may range from 10 to 60 days or from 20 to 40 days such as about 40 days.

Fermentation is carried out in a single fermenter or a single fermentation unit or fermentation facility (50). These may take various forms, such as a concrete or metal tank reactor or an oval reactor. The fermentation unit may also comprise more than one fermenter tank. All steps of the fermentation process occur in a single fermentation reactor; one or more of said fermentation reactors may be present in the system. A further distinction can be made between continuously fed, semi-continuously fed, and batch reactors. In continuously fed reactors, fresh material, in particular the hydrolyzed organic material, is continuously added to the reactors, whereas in semi-continuously fed reactors, the fresh material, in particular the hydrolyzed organic material, is added to the reactor at regular intervals of between about 3 hours and about 6 hours, e.g. every 3, 4, 5 or 6 hour. Preferably, a semi-continuously fed reactor is used in the methods and systems described herein, more preferably the fermentation unit is fed with hydrolysed organic material every 3 to 6 hours, preferably every 4 to 6 hours, such as every 4 hours. In continuously and semi-continuously fed reactors, mixing of fresh and old material in the fermentation unit is necessary so that the microorganisms come into contact with the freshly-fed material more quickly. Mixing can be carried out e.g. by stirring, circulation of the liquid, or by feeding in biogas. The fermenter may be thermoregulated.

The wet end product of fermentation is referred to as the "digestate" (115). The composition of the digestate primarily depends on the composition of the organic material that is fermented. The digestate further contains the mineral nutrients, e.g. inorganic nitrogen and inorganic phosphorous, which are present in the initial organic substrate. The digestate also comprises at least a part of the bacterial biomass contained in the fermenter.

In addition, biogas (117) is produced in the fermentation facility (50). The term "biogas" is understood herein to refer to the gas mixture produced after fermentation of biomass. The biogas primarily comprises methane (CH₄) and carbon dioxide (CO₂), and can further comprise one or more components selected from the group composed of H₂O, H₂S, and NH₃. The exact composition of the biogas is determined by the composition of the organic material that is fermented.

The biogas can be converted into electricity, heat, mechanical energy or a combination of two or more thereof. In addition, it is also possible, optionally after purifying the biogas into biomethane, to impart value to it as a renewable-source natural gas. The bio-natural gas can e.g. be injected into the natural gas network, or it can be compressed at a pressure of 200 to 250 bar into bio-CNG (compressed natural gas), more precisely referred to as CBG (compressed biogas) as well as CBM (compressed biomethane). Biomethane, preferably purified biomethane, can also be used as a precursor for the production of biopolymers.

In embodiments, the produced biogas (117) is cooled in a cooling unit, preferably a condenser, to remove water and H₂S, and said water comprising H₂S is transferred to a photo-oxidation unit for the conversion of H₂S into H₂SO₄. Said H₂SO₄ can advantageously be used to improve the recovery of nitrogen (N) from the organic substrate in the dehydration step as described elsewhere herein.

Biogas production is directly proportional to the anaerobic digestion efficiency and the number of bacteria in the fermenter. In the methods and systems described herein, digestion efficiency is increased amongst others due to the recovery of bacterial biomass from the digestate and its re-introduction in the fermenter, which will also result in increased biogas production.

### Separation of the digestate

In a separator or separating unit (60), the solid (118) and liquid (119) fractions of the digestate are separated from each other, preferably by means of a mechanical solid-liquid separator (60). A variety of solid-liquid separation technologies are available on the market including, without limitation, (decanter) centrifuges, screw press separators, bow sieves or parabolic screens, double circle bow sieves, sieve belt presses, and sieve drum presses. Preferably, or a centrifuge (60), more preferably a parabolic screen, is used in the methods and systems described herein.

The term "solid or thick fraction of the digestate" (118) is understood herein to refer to the fraction of the digestate after separation that contains at least 15% (w/w) of dry matter. This solid or thick fraction comprises undigested and/or partially digested organic matter. This solid fraction is recirculated to the hydrolysis unit (30) for thermal hydrolysis, e.g. by means of a recirculation pump, to allow further hydrolysis of the organic matter. Also the actual residence time of this solid fraction is much greater than the more rapidly digestable fraction of the organic substrate, allowing for a better degradation thereof, thereby increasing digestion efficiency.

The term "liquid or thin fraction of the digestate" (119) is understood herein to refer to the fraction of the digestate after separation that comprises below 6% (w/w) dry matter. The liquid fraction of the digestate contains the greatest part of bacterial biomass from the fermenter. This liquid fraction is sent to the (ultra)filtration step in the method described herein.

Nutrients or inorganic or mineral matter that is present in the organic substrate are not distributed in the same manner between the two fractions after separation, e.g. inorganic phosphorus largely goes to the solid fraction and inorganic nitrogen and potassium can mostly be found in the liquid fraction.

This liquid-solid separation step is also advantageous for the subsequent (ultra)filtration step in that it clarifies the digestate, allowing for a smoother (ultra)filtration. Indeed, without said separation step, membranes can be damaged due to the presence of unseparated dry matter, and the presence of said unseparated dry matter would also increase the viscosity of the digestate, which would in turn increase the power consumption of the filtration process.

To improve solid-liquid separation, e.g. for high-fibre organic substrates, flocculation or precipitation agents such as polyelectrolytes or polymers may be applied.

### Filtration

In the filtration step, the liquid fraction (119) of the digestate is further separated, typically with a smaller separation size than the first separation step, or filtered by passing the liquid digestate (119) through a membrane. Depending on the pore size of the membrane and the trans-membrane pressure, some particles are retained by the membrane, while other, smaller particles, and the relatively purified water permeate pass through the membrane.

Microfiltration can separate particles of 0.1 µm, while ultrafiltration can separate particles and colloids of diameters lower than 0.01 µm. In preferred embodiments, the liquid fraction of the digestate is separated or filtered by microfiltration or ultrafiltration means, preferably ultrafiltration means. In this way, a first stream of filtered liquid digestate (123) substantially free of bacterial biomass flows out from the filtration unit; on the other hand, a retained stream (121) in which the bacterial biomass is concentrated, is retained.

The "filtered fraction" or "filtrate" or "permeate" (123) as used herein refers to the fraction of the liquid fraction (119) of the digestate after filtration that passes through the membrane. This fraction is substantially free of bacterial biomass. In embodiments, the dry matter content of the filtrate is less than 4% (w/w), preferably less than 3% (w/w) such as about 2% (w/w).

The "retained fraction" or "retentate" (121) as used herein refers to the fraction of the liquid fraction (119) of the digestate after filtration that is retained on the membrane. This retained fraction (121) is concentrated in bacterial biomass from the fermenter.

Any type of filtration unit (70) can be used in the methods and systems described herein, including cross-flow filtration units, in which the feed flow travels tangentially across the membrane surface, rather than into the membrane, or rotation filtration units, in which the cross-flow effect is generated by rotating the membranes or by rotating the rotor. Preferably, the filtration unit (70) is a rotation filtration unit.

In the methods described herein, the retained fraction (121) is returned to the anaerobic fermenter (50) by a recirculating stream, preferably moved by a pump. Recycle of the retentate (121) is particularly desirable since it drastically reduces the outflow of fermenting bacteria. The fermenter bacterial density is thereby increased, allowing a more efficient degradation of the organic material and a greater production of biogas. Recycling of the retentate (121) is particularly advantageous when the fermentation occurs in a single fermentation reactor. In contrast, when using multiple fermentation reactors for the different steps of the fermentation process (i.e. hydrolysis of the substrate and acidogen digestion, acetogen digestion and methanogen digestion) with optimal operating conditions for the particular bacteria involved, the digestate will mainly contain bacterial biomass from the last reactor. Recycling of the retentate to the fermenters may hence result in an increased bacterial density in the last reactor, but is unlikely to be beneficial in the other reactors due to the different operating conditions. Accordingly, in the first reactors, there will still be washout of the bacterial biomass.

### Dehydration of the filtrate

In the methods according to the invention, nutrients, particularly nitrogen (N), phosphorus (P) and potassium (K), are recovered from the organic substrate. For this purpose, a dehydration process is applied to the filtrate (123), thereby increasing the nutrient concentration. Preferably, the heat required for said dehydration step comes from residual heat from other process steps such as the generation of electricity from biogas.

In the methods described herein, the dehydration water (127) is recycled for diluting the incoming feedstock.

In some embodiments, nutrients are separated from the filtrate (123) by evaporation. The non-volatile nutrients present in the filtrate are concentrated by evaporating the water (127) and the concentrate (125) is recovered. The vapour is condensed and used for diluting the fresh feedstock.

For the recovery of nitrogen (N) by evaporation, a strongly acidic solution, particularly a sulphuric acid solution, can be added to the filtrate (123) before evaporation. Specifically, nitrogen (N) is converted to volatile ammonia (NH₃) in the fermentation process, which then disappears in the vapour phase on evaporation of the filtrate. Addition of the sulphuric acid solution (H₂SO₄) causes ammonium sulphate ((NH₄)₂SO₄) to be formed, which remains as a residue together with the other non-volatile nutrients after evaporation. As an alternative, the sulphuric acid (H₂SO₄) can be added to the vapour phase after evaporation (i.e. acidic water or gas scrubbing) in order to produce ammonium sulphate ((NH₄)₂SO₄).

Several evaporator (80) types are on the market for use in digester plants, including falling film evaporators, forced circulation evaporators, vacuum evaporators. Preferably, a falling film evaporator is used in the methods and systems described herein.

An alternative technology for separating nutrients from the filtrate is steam stripping, in which steam is blown through the filtrate so that volatile nutrients (e.g. ammonia (NH3)) enter the gas phase. After cleaning of the gas phase, e.g. using an acidic gas or air scrubber, with sulphuric acid (H₂SO₄), nitrogen (N) can therefore be separated in the form of ammonium sulphate ((NH₄)₂SO₄) from the filtrate.

### Drying of the concentrated filtrate

Optionally, the concentrated filtrate (125) obtained by dehydration or evaporation may be dried. The drying process aims to further stabilize the (bio)fertilizer product (129) as well as to further reduce its total mass. It also increases the nutrient concentration while reducing moisture content, to make storage and transportation easier. In embodiments the dry matter content of said dried concentrated filtrate (129) is higher than 50% (w/w), preferably higher than 55% (w/w), more preferably higher than 60% (w/w). This fraction is also enriched in mineral nutrients, in particular nitrogen (N), phosphor (P) and potassium (K), and thus particularly useful as (bio)fertilizer.

Non-limiting examples of suitable dryers (90) are paddle dryer, belt dryer, fluidized bed dryer, feed-and-turn dryer, and sand bed dryer. In embodiments, a paddle dryer is used to dry the concentrated filtrate.

Preferably, the heat for said drying step may be provided by the system described herein, in particular residual heat from engines in the system.

In embodiments, the concentrated filtrate (125) is dried together with matter (111) that has deposited in the hydrolysis units, in particular the thermal hydrolysis unit (30). As described elsewhere herein, the solid fraction of the digestate (118), which also contains mineral matter (e.g. inorganic phosphorus), is recycled to one of the hydrolysis units, preferably the thermal hydrolysis unit (30), for further hydrolysis and anaerobic digestion. The mineral matter contained in said solid fraction (118) can deposit in said (thermal) hydrolysis unit (30) and be discharged through an outlet for drying with the concentrated filtrate (125).

Another aspect of the present invention relates to a system suitable for treating organic material according to the methods described herein. In particular, a system (100) is provided comprising:
a) one or more hydrolysis units (10, 20, 30 40) for hydrolyzing the organic material (101) by mechanical hydrolysis, chemical hydrolysis, thermal hydrolysis and biological hydrolysis;
b) a single anaerobic fermenter (50) for the production of biogas (11) and digestate (115) by fermentation of the hydrolyzed organic material (113), said fermenter being fed with the hydrolyzed organic material (113) and comprising at least two output conduits from which the biogas (117) and the digestate (115) are discharged;
c) a separator (60) connected to the fermenter (50) via the output conduit and receiving the digestate (115), said separator (60) being capable of separating the digestate (115) into a liquid fraction (119) and a solid fraction (118);
d) a conduit allowing said solid fraction (118) of the digestate to flow out of said separator (60) and to be returned to the hydrolysis unit for hydrolyzing the organic material (101) by thermal hydrolysis (30);
e) a filtration system (70) for separating the liquid fraction (119) of the digestate into a filtrate (123) and a retentate (121), said filtration system (70) comprising a membrane through which the liquid fraction (119) of the digestate is filtered to obtain a filtrate (123) and a retentate (121) containing bacterial biomass;
f) a conduit allowing the retentate (121) to flow out of said filtration system (70) and to be returned to the anaerobic fermenter (50);
g) a dehydration device (80) for concentrating the filtrate (123), wherein said dehydration device (80) is connected to the filtration system (70) through a conduit allowing the filtrate (123) to flow out of the filtration system (70);
h) a conduit allowing the dehydration water (127) from the dehydration device (80) to recycle to the hydrolysis unit (20); and
optionally i) a dryer (90) for drying the concentrated filtrate (125).

### EXAMPLES

### Example 1

An embodiment of the invention is discussed with reference to Fig. 1. An organic substrate (101) rich in biodegradable organic matter (at least 80% of the dry matter content), is subjected to mechanical hydrolysis in a mechanical hydrolysis unit (10) comprising a hopper with mortar or push floor, and a hammer mill to fractionate the organic substrate by applying physical forces. The mechanically hydrolyzed substrate (103) is diluted with water (127) originating from the evaporation unit (80) and then further hydrolyzed by consecutively chemical, thermal and biological hydrolysis. The chemical hydrolysis unit (20) comprises a concrete tank equipped with multiple mixers for mixing the organic substrate with an alkali reagent, e.g. lime, which is added until pH 8.5. The chemical hydrolysis unit (20) further comprises an outlet (107) for discharging deposited material. The mix (105) from the chemical hydrolysis, which is at pH 8.5, is then pumped to a thermal hydrolysis unit (30). This thermal hydrolysis unit (30) is a tank with conical bottom, which is thermoregulated at about 85°C using residual heat from engines in the system. The tank is equipped with a top entry mixer. The residence time of the substrate in the tank is at least 1 hour. An outlet situated at the conical bottom allows to discharge deposited inert material (111) from the system. The mechanically, chemically and thermal hydrolyzed substrate (109) is then transferred to a biological hydrolysis unit (40), where it is retained for a couple of days, typically about 72 hours, to further hydrolyse the substrate by microbial action. The biological hydrolysis unit (40) is a tank with a volume of about 1500 m³ made from a material, e.g. enamelled metal, that is resistant to low pH (e.g. pH 5). The tank is cooled, and the reactor content is mixed by an external mixer.

Following the hydrolysis of the substrate, it is pumped to the fermentation unit (50) comprising 4 fermenter tanks, each having a volume of about 5000 m³. The fermenters are continuously fed. The content of the fermenters is mixed with 3 different external mixers. The hydraulic retention time is about 40 days. The hydrolyzed substrate (113) is converted into biogas (117) and a digestate (115) in said fermentation unit (50).

The wet digestate (115) is separated in a centrifuge (60) into a liquid (119) and a solid (118) fraction.

The solid fraction (118), which contains undigested and partially digested organic matter and mineral matter, is through a conduit recycled to the thermal hydrolysis unit (30) for further hydrolysis and fermentation. The mineral matter (111) contained in said solid fraction can deposit in the conical bottom of said tank (30) and be discharged from the system through an outlet.

The liquid fraction (119) of the digestate is filtered in an ultrafiltration unit (70) into a filtrate (123) and a retentate (121).

The retained fraction (121) with bacterial biomass is returned to the anaerobic fermenter (50) through a recirculation conduit.

The fitrate (123) is after addition of sulphuric acid concentrated into an evaporator (80). This concentrate (125) is, together with deposited matter (111) from the thermal hydrolysis unit (30), further dried into a paddle dryer (90) to produce a dry (bio)fertilizer (129) with a dry matter content higher than 60% (w/w) and enriched in mineral nutrients, in particular inorganic nitrogen (N), inorganic phosphorus (P) and inorganic potassium (K). The vapour from the evaporation unit (80) is condensed and this evaporation water (127) is used for diluting the organic substrate (101).

## Claims

1. A process for the treatment of organic material, comprising the following steps:
(a) hydrolyzing the organic material by mechanical hydrolysis, chemical hydrolysis, thermal hydrolysis and biological hydrolysis;
(b) anaerobic digestion of the hydrolyzed organic material in a single fermenter (50) into biogas and digestate;
(c) separating the digestate into a liquid fraction and a solid fraction by mechanical means;
(d) recycling the solid fraction of the digestate to the thermal hydrolysis;
(e) separating the liquid fraction of the digestate into a filtrate and a retentate containing bacterial biomass by filtration means;
(f) recycling the retentate containing bacterial biomass to the fermenter;
(g) concentrating the filtrate by dehydration; and
(h) recycling of the dehydration water from step (g) to the hydrolysis step (a) for diluting the organic material.

2. The process according to claim 1, wherein said process further comprises a step (i) of drying the concentrated filtrate.

3. The process according to claim 1 or 2, wherein an alkali reagent, preferably lime (CaO), is used for said chemical hydrolysis.

4. The process according to claim 1 or 2, wherein said thermal hydrolysis is conducted at a temperature above 70°C for at least 1 hour.

5. The process according to claim 1 or 2, wherein said biological hydrolysis is conducted by microbial organisms present in the organic material.

6. The process according to any one of claims 1 to 5, wherein said separation step (c) comprises the centrifugation of the digestate or wherein a parabolic screen is used for said separation step (c), preferably a parabolic screen is used.

7. The process according to any one of claims 1 to 6, wherein the filtrate is concentrated in step (g) by evaporation.

8. A system (100) for the treatment of organic material (101), comprising:
a) one or more hydrolysis units (10, 20, 30, 40) for hydrolyzing the organic material (101) by mechanical hydrolysis, chemical hydrolysis, thermal hydrolysis and biological hydrolysis;
b) a single anaerobic fermenter (50) for the production of biogas (117) and digestate (115) by fermentation of the hydrolyzed organic material (113), said fermenter being fed with the hydrolyzed organic material (113) and comprising at least two output conduits from which the biogas (117) and the digestate (115) are discharged;
c) a separator (60) connected to the fermenter via the output conduit and receiving the digestate (115), said separator (60) being capable of separating the digestate (115) into a liquid fraction (119) and a solid fraction (118);
d) a conduit allowing said solid fraction of the digestate (118) to flow out of said separator (60) and to be returned to the hydrolysis unit for hydrolyzing the organic material (101) by thermal hydrolysis (30);
e) a filtration system (70) for separating the liquid fraction (119) of the digestate into a filtrate (123) and a retentate (121), said filtration system (70) comprising a membrane through which the liquid fraction of the digestate (119) is filtered to obtain a filtrate (123) and a retentate (121) containing bacterial biomass;
f) a conduit allowing the retentate (121) to flow out of said filtration system (70) and to be returned to the anaerobic fermenter (50);
g) a dehydration device (80) for concentrating the filtrate (123), wherein said dehydration device (80) is connected to the filtration system (70) through a conduit allowing the filtrate (123) to flow out of the filtration system (70);
h) a conduit allowing the dehydration water (127) from the dehydration device (80) to recycle to the hydrolysis unit (20).

9. The system according to claim 8, further comprising:
i) a dryer (90) for drying the concentrated filtrate (125).

10. The system according to any one of claims 8 or 9, wherein said separator (60) is a centrifuge or a parabolic screen, preferably a parabolic screen.

11. The system according to any one of claims 8 to 10, wherein said filtration system (70) is an ultrafiltration system.

12. The system according to any one of claims 8 to 11, wherein said dehydration device (80) is an evaporator.

## Patentansprüche

1. Verfahren zur Behandlung von organischem Material, umfassend die folgenden Schritte:
(a) Hydrolysieren des organischen Materials durch mechanische Hydrolyse, chemische Hydrolyse, thermische Hydrolyse und biologische Hydrolyse;
(b) anaerober Aufschluss des hydrolysierten organischen Materials in einem einzigen Fermenter (50) zu Biogas und Gärrest;
(c) Auftrennen des Gärrests in eine flüssige Fraktion und eine feste Fraktion durch mechanische Mittel;
(d) Rückführen der festen Fraktion des Gärrests zu der thermischen Hydrolyse;
(e) Auftrennen der flüssigen Fraktion des Gärrests in ein Filtrat und ein Retentat, das bakterielle Biomasse enthält, durch Filtrationsmittel;
(f) Rückführen des Retentats, das bakterielle Biomasse enthält, in den Fermenter;
(g) Konzentrieren des Filtrats durch Dehydratation; und
(h) Rückführen des Dehydratationswassers aus Schritt (g) zu dem Hydrolyseschritt (a) zum Verdünnen des organischen Materials.

2. Verfahren gemäß Anspruch 1, wobei das Verfahren ferner einen Schritt (i) des Trocknens des konzentrierten Filtrats umfasst.

3. Verfahren gemäß Anspruch 1 oder 2, wobei ein alkalisches Reagens, vorzugsweise Kalk (CaO), für die chemische Hydrolyse verwendet wird.

4. Verfahren gemäß Anspruch 1 oder 2, wobei die thermische Hydrolyse bei einer Temperatur von über 70 °C für wenigstens 1 Stunde durchgeführt wird.

5. Verfahren gemäß Anspruch 1 oder 2, wobei die biologische Hydrolyse durch in dem organischen Material vorhandene mikrobielle Organismen durchgeführt wird.

6. Verfahren gemäß einem der Ansprüche 1 bis 5, wobei der Trennschritt (c) die Zentrifugation des Gärrests umfasst oder wobei ein parabolisches Sieb für den Trennschritt (c) verwendet wird, wobei vorzugsweise ein parabolisches Sieb verwendet wird.

7. Verfahren gemäß einem der Ansprüche 1 bis 6, wobei das Filtrat bei Schritt (g) durch Verdunstung konzentriert wird.

8. System (100) zur Behandlung von organischem Material (101), umfassend:
a) eine oder mehrere Hydrolyseeinheiten (10, 20, 30, 40) zum Hydrolysieren des organischen Materials (101) durch mechanische Hydrolyse, chemische Hydrolyse, thermische Hydrolyse und biologische Hydrolyse;
(b) einen einzigen anaeroben Fermenter (50) für die Herstellung von Biogas (117) und Gärrest (115) durch Fermentation des hydrolysierten organischen Materials (113), wobei der Fermenter mit dem hydrolysierten organischen Material (113) gespeist wird und wenigstens zwei Ausgangsleitungen umfasst, aus denen das Biogas (117) und der Gärrest (115) abgeführt werden;
(c) einen Separator (60), der über die Ausgangsleitung mit dem Fermenter verbunden ist und den Gärrest (115) erhält, wobei der Separator (60) fähig ist, den Gärrest (115) in eine flüssige Fraktion (119) und eine feste Fraktion (118) aufzutrennen;
(d) eine Leitung, die erlaubt, die feste Fraktion des Gärrests (118) aus dem Separator (60) abfließen zu lassen und zu der Hydrolyseeinheit zum Hydrolysieren des organischen Materials (101) durch thermische Hydrolyse (30) zurückzuführen;
(e) ein Filtrationssystem (70) zum Auftrennen der flüssigen Fraktion (119) des Gärrests in ein Filtrat (123) und ein Retentat (121), wobei das Filtrationssystem (70) eine Membran umfasst, durch die die flüssige Fraktion des Gärrests (119) filtriert wird, um ein Filtrat (123) und ein Retentat (121), das bakterielle Biomasse enthält, zu erhalten;
(f) eine Leitung, die erlaubt, das Retentat (121) aus dem Filtrationssystem (70) abfließen zu lassen und zu dem anaeroben Fermenter (50) zurückzuführen;
(g) eine Dehydratationsvorrichtung (80) zum Konzentrieren des Filtrats (123), wobei die Dehydratationsvorrichtung (80) durch eine Leitung, die Abfließen des Filtrats (123) aus dem Filtrationssystem (70) erlaubt, mit dem Filtrationssystem (70) verbunden ist;
(h) eine Leitung, die Rückführen des Dehydratationswassers (127) von der Dehydratationsvorrichtung (80) zu der Hydrolyseeinheit (20) erlaubt.

9. System gemäß Anspruch 8, ferner umfassend:
i) einen Trockner (90) zum Trocknen des konzentrierten Filtrats (125).

10. System gemäß einem der Ansprüche 8 oder 9, wobei der Separator (60) eine Zentrifuge oder ein parabolisches Sieb, vorzugsweise ein parabolisches Sieb, ist.

11. System gemäß einem der Ansprüche 8 bis 10, wobei das Filtrationssystem (70) ein Ultrafiltrationssystem ist.

12. System gemäß einem der Ansprüche 8 bis 11, wobei die Dehydratationsvorrichtung (80) ein Verdampfer ist.

## Revendications

1. Procédé pour le traitement d'un matériau organique, comprenant les étapes suivantes :
(a) hydrolyse du matériau organique par hydrolyse mécanique, hydrolyse chimique, hydrolyse thermique et hydrolyse biologique ;
(b) digestion anaérobie du matériau organique hydrolysé dans un seul fermenteur (50) en biogaz et digestat ;
(c) séparation du digestat en une fraction liquide et une fraction solide par des moyens mécaniques ;
(d) recyclage de la fraction solide du digestat vers l'hydrolyse thermique ;
(e) séparation de la fraction liquide du digestat en un filtrat et un rétentat contenant de la biomasse bactérienne par des moyens de filtration ;
(f) recyclage du rétentat contenant de la biomasse bactérienne vers le fermenteur ;
(g) concentration du filtrat par déshydratation ; et
(h) recyclage de l'eau de déshydratation issue de l'étape (g) vers l'étape d'hydrolyse (a) pour diluer le matériau organique.

2. Procédé selon la revendication 1, ledit procédé comprenant en outre une étape (i) de séchage du filtrat concentré.

3. Procédé selon la revendication 1 ou 2, dans lequel un réactif alcalin, de préférence de la chaux (CaO), est utilisé pour ladite hydrolyse chimique.

4. Procédé selon la revendication 1 ou 2, dans lequel ladite hydrolyse thermique est conduite à une température supérieure à 70 °C pendant au moins 1 heure.

5. Procédé selon la revendication 1 ou 2, dans lequel ladite hydrolyse biologique est conduite par des organismes microbiens présents dans le matériau organique.

6. Procédé selon l'une quelconque des revendications 1 à 5, dans lequel ladite étape de séparation (c) comprend la centrifugation du digestat ou dans lequel un tamis parabolique est utilisé pour ladite étape de séparation (c), de préférence un tamis parabolique est utilisé.

7. Procédé selon l'une quelconque des revendications 1 à 6, dans lequel le filtrat est concentré à l'étape (g) par évaporation.

8. Système (100) pour le traitement d'un matériau organique (101), comprenant :
a) une ou plusieurs unités d'hydrolyse (10, 20, 30, 40) pour hydrolyser le matériau organique (101) par hydrolyse mécanique, hydrolyse chimique, hydrolyse thermique et hydrolyse biologique ;
b) un seul fermenteur anaérobie (50) pour la production de biogaz (117) et digestat (115) par fermentation du matériau organique hydrolysé (113), ledit fermenteur étant alimenté avec le matériau organique hydrolysé (113) et comprenant au moins deux conduites de sortie à partir desquelles le biogaz (117) et le digestat (115) sont évacués ;
c) un séparateur (60) raccordé au fermenteur par la conduite de sortie et recevant le digestat (115), ledit séparateur (60) pouvant séparer le digestat (115) en une fraction liquide (119) et une fraction solide (118) ;
d) une conduite permettant à ladite fraction solide du digestat (118) de s'écouler hors dudit séparateur (60) et d'être retournée à l'unité d'hydrolyse pour hydrolyser le matériau organique (101) par hydrolyse thermique (30) ;
e) un système de filtration (70) pour séparer la fraction liquide (119) du digestat en un filtrat (123) et un rétentat (121), ledit système de filtration (70) comprenant une membrane à travers laquelle la fraction liquide du digestat (119) est filtrée pour obtenir un filtrat (123) et un rétentat (121) contenant de la biomasse bactérienne ;
f) une conduite permettant au rétentat (121) de s'écouler hors dudit système de filtration (70) et d'être retourné au fermenteur anaérobie (50) ;
g) un dispositif de déshydratation (80) pour concentrer le filtrat (123), ledit dispositif de déshydratation (80) étant raccordé au système de filtration (70) par une conduite permettant au filtrat (123) de s'écouler hors du système de filtration (70) ;
h) une conduite permettant à l'eau de déshydratation (127) issue du dispositif de déshydratation (80) d'être recyclée vers l'unité d'hydrolyse (20).

9. Système selon la revendication 8, comprenant en outre :
i) un séchoir (90) pour sécher le filtrat concentré (125) .

10. Système selon l'une quelconque des revendications 8 et 9, dans lequel ledit séparateur (60) est une centrifugeuse ou un tamis parabolique, de préférence un tamis parabolique.

11. Système selon l'une quelconque des revendications 8 à 10, dans lequel ledit système de filtration (70) est un système d'ultrafiltration.

12. Système selon l'une quelconque des revendications 8 à 11, dans lequel ledit dispositif de déshydratation (80) est un évaporateur.
